(19) Europäisches Patentamt
European Patent Office
Office européen des brevets

(11) **EP 4 506 789 A1**

(12) **EUROPEAN PATENT APPLICATION**
published in accordance with Art. 153(4) EPC

(43) Date of publication:
**12.02.2025 Bulletin 2025/07**

(21) Application number: **23910273.4**

(22) Date of filing: **19.12.2023**

(51) International Patent Classification (IPC):
**G06F 3/01** (2006.01)

(52) Cooperative Patent Classification (CPC):
Y02D 10/00

(86) International application number:
**PCT/CN2023/139927**

(87) International publication number:
**WO 2024/140343 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **29.12.2022 CN 202211705807**

(71) Applicant: **Kingfar International Inc.**
**100085 Beijing (CN)**

(72) Inventors:
• **ZHAO, Qichao**
 **Beijing 100085 (CN)**
• **YANG, Ran**
 **Beijing 100085 (CN)**

(74) Representative: **Dr. Gassner & Partner mbB**
**Wetterkreuz 3**
**91058 Erlangen (DE)**

(54) **GAZE-TRAJECTORY-BASED EYE MOVEMENT DATA VISUALIZATION METHOD AND APPARATUS, AND STORAGE MEDIUM**

(57) A gaze-trajectory-based eye movement data visualization method and apparatus, and a storage medium. The method comprises: by means of collected eye movement data, obtaining a tested gaze point position, gaze point sequence, and gaze point trajectory (S110); according to the angle of a connecting line between two neighboring gaze points, mapping the connecting line between the two neighboring gaze points into a visualization graph (S130); and within the visualization graph, connecting the vertex of the connecting line corresponding to each angle, forming a visualization graph of eye movement glancing direction frequency (S140). The visualization graph of eye movement glancing direction frequency is used to provide an eye movement gaze trajectory algorithm, allowing for visually and accurately reflecting individual eye movement gaze features.

collecting eye movement data of a subject, and based on the collected eye movement data, obtaining gaze point position, gaze point sequence, and gaze point trajectory of the subject; — S110

determining a positional relationship between adjacent gaze points according to the gaze point sequence in the gaze point trajectory, connecting every two adjacent gaze points, and determining degree of an included angle between the line connecting the two adjacent gaze points and a predetermined reference axis; — S120

based on the degree of an included angle between the line connecting two adjacent gaze points and the predetermined reference axis, mapping the line connecting the two adjacent gaze points to a visualization graph via the included angle, wherein, when mapping, if there are already a line with the same included angle mapped on the visualization graph, the line connecting the two adjacent gaze points extends along the line with the same angle mapped on the visualization graph by a unit length; and — S130

forming the visualization graph of a frequency of eye movement scanning direction by connecting vertices of extended lines corresponding to individual angles in the visualization graph — S140

FIG. 1

EP 4 506 789 A1

## Description

### RELATED APPLICATION

The present application claims priority to China patent application serial number CN202211705807.6, titled "Eye Movement Data Visualization Method, Device, and Storage Medium Based on Gaze Trajectory", filed with China Patent Office on December 29, 2022. The entirety of this application is incorporated herein by reference.

### TECHNICAL FIELD

The present application relates to the field of eye movement data analysis technology, and, more particularly to a method, a device, and a storage medium for visualizing eye movement data based on gaze trajectories.

### BACKGROUND ART

Vision is an important way for humans to perceive and obtain external things and information. In this process, dynamic visual perception of surrounding things is mainly achieved through the movement of the eyes. Therefore, eye movement can reveal human visual mechanisms and cognitive activities. Studying human eye movement can effectively understand person's intentions when observing different things, and can be used for usability evaluation of products such as images and videos. eye movement technology obtains raw data of user's eye movements through eye trackers, and based on the raw data, obtains user's visual characteristics, and then analyzes user's behavioral data such as preferences when using products, providing data references for improving products, eye movement technology has been widely applied in fields such as interface design, human-computer interaction, and reading habit analysis.

By using eye movement technology to obtain the user's raw eye movement data, eye movement data analysis can be conducted based on the raw eye movement data to obtain the user's visual characteristics on the observed object, such as eye movement gaze position, gaze time, gaze frequency, gaze diameter, etc. Through visualization processing, eye movement visualization images such as eye heat maps, eye movement trajectory maps, 3D maps, etc. can be obtained to obtain the user's visual characteristics when viewing the images. However, the results obtained from the above eye movement visualization are only based on the analysis of eye movement gaze features, resulting in single indicator visualization.

### SUMMARY

In view of this, the embodiments of the present application provide a gaze trajectory based eye movement data visualization method, device, and storage medium to eliminate or improve one or more deficiencies existing in the prior art.

One aspect of the present application provides an eye movement data visualization method based on gaze trajectory, including the following steps:
collecting eye movement data of a subject, and based on the collected eye movement data, obtain gaze point position, gaze point sequence, and gaze point trajectory of the subject; determining the positional relationship between adjacent gaze points according to the gaze point sequence in the gaze point trajectory, connect every two adjacent gaze points by a line, and determine the angle between the line connecting the two adjacent gaze points and the predetermined reference axis; and based on the included angle between the line connecting two adjacent gaze points and a predetermined reference axis, mapping the line connecting the two adjacent gaze points to a visualization graph at the included angle. When mapping, if there are already lines with the same included angle mapped on the visualization graph, the line connecting the two adjacent gaze points extends along the line with the same angle mapped on the visualization graph by a unit length. By connecting the vertices of the extended lines corresponding to individual angles in the visualization graph, a visualization graph of the frequency of eye movement scanning direction is formed.

In some embodiments of the present application, the step of determining the angle between the line connecting two adjacent gaze points and a predetermined reference axis includes: establishing a plane Cartesian coordinate system with the previous gaze point as the origin, determining the position coordinate of the latter gaze point in the plane Cartesian coordinate system, and then determining the angle between the line connecting two adjacent gaze points and the predetermined coordinate axis in the plane Cartesian coordinate system.

In some embodiments of the present application, the step of determining the angle between the line connecting two adjacent gaze points and the predetermined coordinate axis in the plane Cartesian coordinate system comprises: based on the plane Cartesian coordinate system with the previous gaze point as the origin, determining the quadrant coordinates of the latter gaze point in the plane Cartesian coordinate system, and determining the degree of counterclockwise angle between the line connecting two adjacent gaze points and the X-axis of the plane Cartesian coordinate system.

In some embodiments of the present application, the step of determining the degree of the included angle between the line connecting two adjacent gaze points and the X-axis of the plane Cartesian coordinate system counterclockwise includes: converting the quadrant coordinate of the last gaze point in every two adjacent gaze points into the coordinate of the first quadrant through axis symmetry or center symmetry, and measuring the degree of the included angle between the line connecting the two adjacent gaze points after the conversion of the last gaze point into the quadrant and the X-axis of the plane Cartesian coordinate system counterclockwise; and based on the quadrant coordinates of the latter gaze point in the plane Cartesian coordinate system, determining the degree of the included angle between the line connecting two adjacent gaze points and the X-axis of the plane Cartesian coordinate system counterclockwise.

In some embodiments of the present application, the formula for calculating the degree of angle between the line connecting two adjacent gaze points after the conversion of one gaze point into a quadrant and the X-axis of the plane Cartesian coordinate system counterclockwise is:

$$\text{Arctan}\theta' = (|y_2|-y_1)/(|x_2|-x_1)$$

where the previous gaze point is used as the first eye movement gaze point and the subsequent gaze point is used as the second eye movement gaze point, wherein $\theta'$ is the degree of the included angle between the line connecting two adjacent gaze points after measuring the conversion quadrant of one gaze point and the X-axis of the plane Cartesian coordinate system counterclockwise; $X_1$ is the horizontal ordinate of the first eye movement gaze point; $X_2$ is the horizontal ordinate of the second eye movement gaze point; Yi is the vertical ordinate of the first eye movement gaze point; $Y_2$ is the vertical ordinate of the second eye movement gaze point; $|Y_2|$ is the vertical coordinate of the second eye movement gaze point after being converted into the first quadrant; and $|X_2|$ is the horizontal coordinate of the second eye movement gaze point after being converted into the first quadrant.

In some embodiments of the present application, the step of determining the degree of the included angle between the line connecting two adjacent gaze points and the X-axis of the plane Cartesian coordinate system counterclockwise based on the quadrant coordinates of the latter gaze point in the plane Cartesian coordinate system is as follows: when the latter gaze point is located in the first quadrant, the obtained degree of the included angle is $\theta'$; when the coordinates of the latter gaze point are within the second quadrant, the obtained degree of the included angle is $180° - \theta'$; when the coordinates of the latter gaze point are located in the third quadrant, the obtained degree of the included angle is $180° + \theta'$; and when the coordinates of the latter gaze point are located in the fourth quadrant, the resulting degree of the included angle is $360° - \theta'$.

In some embodiments of the present application, the visualization graph of the frequency of eye movement scanning direction corresponds to the collected eye movement data and is synchronously mapped to form a dynamic visualization graph of the frequency of eye movement scanning direction.

In some embodiments of the present application, the collected eye movement data is collected through an eye tracker.

Another aspect of the present application provides an eye movement data visualization device based on gaze trajectory, including a processor and a memory, wherein the memory stores computer instructions, and the processor is used to execute the computer instructions stored in the memory. When the computer instructions are executed by the processor, the system implements the steps of the eye movement data visualization method as described above.

Another aspect of the present application provides a computer-readable storage medium storing a computer program, wherein the program, when executed by a processor, implements the steps of the eye movement data visualization method as described above.

The eye movement data visualization method and device based on gaze trajectory of the present application display the gaze trajectory of a user observing visual field information in a specific application scenario through visualization graphs. The visualization graphs can intuitively present the visual characteristics when observing visual field information, including gaze point trajectories and the frequency of each gaze point trajectory, in order to judge the rationality of content information design in different application scenarios, effectively optimize the visual field information design in the application scenario, and improve the efficiency of users obtaining visual field information in specific application scenarios.

The additional advantages, objectives, and features of the present application will be partially elaborated in the following description, and will become apparent to those of ordinary skill in the art after studying the following, or may be learned through practice of the present application. The purpose and other advantages of the present application can be achieved and obtained through the structure specifically indicated in the specification and drawings.

Those skilled in the art will understand that the objectives and advantages that can be achieved with the present application are not limited to the specific description above, and based on the following detailed description, they will have a clearer understanding of the above and other objectives that can be achieved with the present application.

**BRIEF DESCRIPTION OF THE DRAWINGS**

The Drawings described here are intended to provide a further understanding of the present application, forming a part of the present application, and not intended to limit the present application. The components in the Drawings are not drawn to scale, but only to illustrate the principles of the present application. For the convenience of illustrating and describing some parts of the present application, corresponding parts in the Drawings may be enlarged, that is, they may become larger relative to other components in the exemplary device actually manufactured according to the present application. In the Drawings:

FIG. 1 shows the visualization process of eye movement data based on gaze trajectory.

FIG. 2 is a flowchart of eye movement data visualization based on gaze trajectory in an embodiment of the present application.

FIGs. 3, 4, 5, and 6 are schematic diagrams of the angle positions between two adjacent gaze point lines and a predetermined reference axis in an embodiment of the present application.

FIG. 7 is a schematic diagram of visualization in an embodiment of the present application.

FIG. 8 shows the visual representation of gaze trajectory and corresponding frequency of eye movement scanning direction.

FIG. 9 is a visual representation of the gaze point trajectory and corresponding eye movement scanning direction frequency in one embodiment of the present application.

FIG. 10 is a structural diagram of an eye movement data visualization device based on gaze trajectory in an embodiment of the present application.

## DETAILED DESCRIPTION

In order to clarify the purpose, technical solution, and advantages of the present application, further detailed explanation of the present application will be provided in conjunction with the embodiments and Drawings. Here, the schematic embodiments and their explanations of the present application are used to explain the present application, but are not intended to limit the present application.

Here, it should be noted that, in order to avoid blurring the present application due to unnecessary details, only the structures and/or processing steps closely related to the solution according to the present application are shown in the Drawings, and other details that are not related to the present application are omitted.

It should be emphasized that the term 'including/containing' when used herein refers to the presence of features, elements, steps, or components, but does not exclude the presence or addition of one or more other features, elements, steps, or components.

Here, it should be noted that unless otherwise specified, the term "connection" herein can not only refer to direct connections, but also to indirect connections with intermediate obj ects.

In the following text, embodiments of the present application will be described with reference to the Drawings. In the Drawings, the same reference numerals represent the same or similar components, or the same or similar steps.

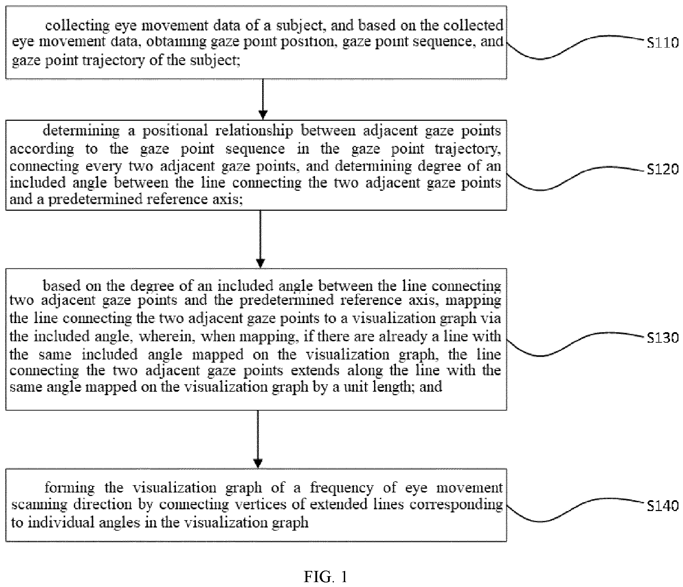

The present application provides a method for visualizing eye movement data based on gaze trajectory, as shown in FIG. 1, including steps S110 to S140:
In step S110, eye movement data of a subject is collected, and based on the collected eye movement data, gaze point position, gaze point sequence, and gaze point trajectory of the subject are obtained.

In one embodiment, the method described in the present application is applied to APP interface design, including: using an eye tracker to collect eye movement data of the subject while browsing the current APP interface, obtaining the gaze point position of the subject's eyes in the APP interface, as well as the gaze point sequence of the subject's eyes changing in the APP interface during the process of browsing the APP interface, and connecting lines according to the gaze point sequentially to obtain gaze point trajectories.

In step S120, the position relationship between adjacent gaze points is determined according to the gaze point sequence in the gaze point trajectory. Every two adjacent gaze points are connected by a line, and the angle between the line connecting the two adjacent gaze points and the predetermined reference axis is determined.

In the above step S120, in the APP interface, a plane Cartesian coordinate system is established with the previous gaze point of every two adjacent gaze points as the origin and the horizontal direction of the APP interface as the X-axis direction, and the X-axis of the plane Cartesian coordinate system is used as the predetermined reference axis. Based on this, the step of determining the angle between the line connecting two adjacent gaze points and the predetermined reference axis includes: establishing a plane Cartesian coordinate system with the previous gaze point as the origin, determining the position coordinate of the latter gaze point in the plane Cartesian coordinate system, and then determining the angle between the line connecting two adjacent gaze points and the predetermined coordinate axis in the plane Cartesian coordinate system.

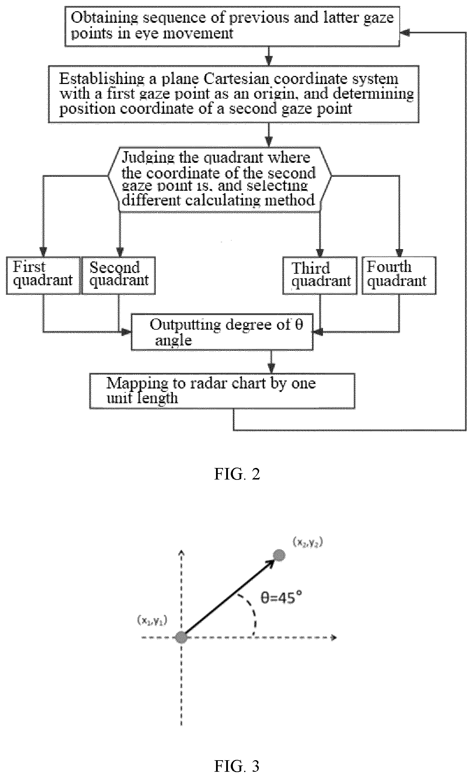

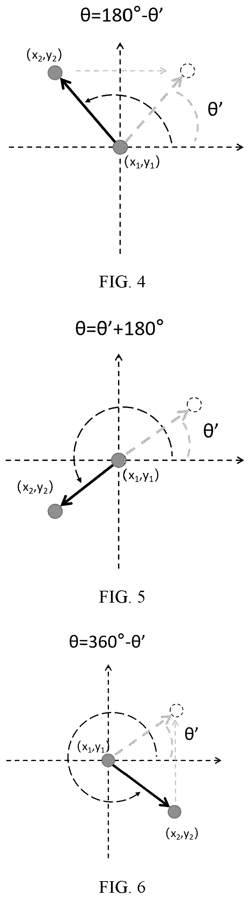

In one embodiment, the step of determining the angle between the line connecting two adjacent gaze points and the predetermined reference axis is shown in FIG. 2: establishing a plane Cartesian coordinate system with the previous gaze point as the origin, determining the position coordinate of the latter gaze point in the plane Cartesian coordinate system, and then determining the angle between the line connecting two adjacent gaze points and the predetermined coordinate axis in the plane Cartesian coordinate system. The calculation step of determining the angle between the line connecting two adjacent gaze points and the predetermined coordinate axis in the plane Cartesian coordinate system includes: determining the quadrant coordinates of a latter gaze point in the plane Cartesian coordinate system, converting the quadrant coordinates of the latter gaze point in every two adjacent gaze points to the coordinates of the first quadrant through axis symmetry or center symmetry, and measuring the degree of the included angle $\theta'$ counterclockwise between the line connecting the two adjacent gaze points after the conversion of the latter gaze point to the X-axis of the plane Cartesian coordinate system. Based on the quadrant coordinates of the latter gaze point in the plane Cartesian coordinate system, the angle θ between the line connecting two adjacent gaze points and the X-axis of the plane Cartesian coordinate system counterclockwise is determined. When the coordinates of the latter gaze point are located in the first quadrant, as shown in FIG. 3, the angle θ between the line connecting the two adjacent gaze points and the X-axis of the plane Cartesian coordinate system counterclockwise is θ'. When the coordinates of the second gaze point are located in the second quadrant, as shown in FIG. 4, the angle θ between the line connecting the two adjacent gaze points and the X-axis of the plane Cartesian coordinate system counterclockwise is 180 ° - θ'. When the coordinates of the latter gaze point are located in the third quadrant, as shown in FIG. 5, the angle θ between the line connecting the two adjacent gaze points and the X-axis of the plane Cartesian coordinate system counterclockwise is 180 °+θ'. When the coordinates of the latter gaze point are located in the fourth quadrant, as shown in FIG. 6, the angle θ between the line connecting the two adjacent gaze points and the X-axis of the plane Cartesian coordinate system counterclockwise is 360 ° - θ'.

In the above embodiment, a formula for calculating the degree of the included angle between the line connecting two adjacent gaze points after the transformation of one gaze point into a quadrant and the counterclockwise X-axis of the plane Cartesian coordinate sys-tem is:

$$\text{Arctan}\theta' = (|y_2|-y_1)/(|x_2|-x_1)$$

A previous gaze point is used as the first eye movement gaze point and a subsequent gaze point is used as the second eye movement gaze point. In particular, θ' is an degree of the included angle between the line connecting two adjacent gaze points after measuring the conversion quadrant of one gaze point and the X-axis of the plane Cartesian coordinate system counterclock-wise. $X_1$ is the horizontal ordinate of the first eye move-ment gaze point. $X_2$ is the horizontal ordinate of the second eye movement gaze point. Yi is the vertical ordinate of the first eye movement gaze point. $Y_2$ is the vertical ordinate of the second eye movement gaze point; $|Y_2|$ is the vertical coordinate of the second eye movement gaze point after being converted into the first quad-rant; and $|X_2|$ is the horizontal coordinate of the second eye movement gaze point after being converted into the first quadrant.

In step S130, based on the included angle between the line connecting two adjacent gaze points and the predetermined reference axis, the line connecting the two adjacent gaze points is mapped to the visualization graph via the degree of the included angle. When mapping, if there are already lines with the same degree of the included angle mapped on the visualization graph, the line connecting the two adjacent gaze points extends on the line with the same degree of the included angle already mapped on the visualization graph by a unit length.

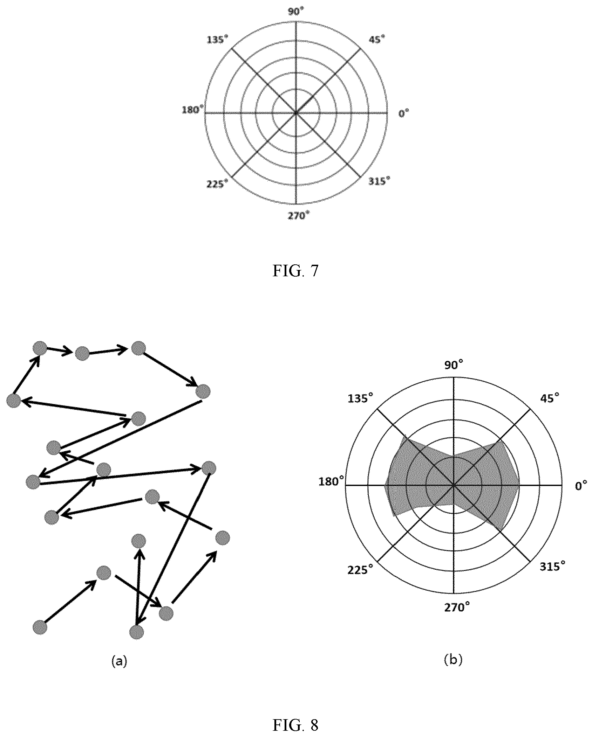

In the above step S130, the visualization graph is displayed through a radar chart, as shown in FIG. 7. Based on this, with real-time eye movement data collected, the line between each two adjacent gaze points is synchronously mapped to the corresponding angle line of the visualization graph according to the included angle between the line and the predetermined reference axis. With the real-time collection of eye movement data, the line between two adjacent gaze points is synchronously mapped to the corresponding angle of the visualization graph. If the angle at which the line between the current two adjacent gaze points is mapped to the visualization graph is the same as the angle at which the line between the previously collected two adjacent gaze points is mapped to the visualization graph, then the line corresponding to that angle of the visualization graph is extended by one unit length from the center point of the visualization graph towards the outer measurement direction.

In one embodiment, the step of mapping the line between every two adjacent gaze points to the corresponding angle position of the visualization graph based on the included angle between the line between two adjacent gaze points and the predetermined reference axis, as shown in FIG. 2, includes: mapping the line between two adjacent gaze points to the corresponding angle position of the visualization graph at a unit length according to the degree of the included angle between the line between the two adjacent gaze points and the predetermined reference axis. With the real-time collection of eye movement data, steps S 120 and S120 above are repeated, and the lines between two adjacent gaze points collected in the future is mapped to the visualization graph by the same calculation method according to the gaze point order of the gaze trajectory. In the case where there are already lines with the same angle and angle mapped on the visualization graph, the lines between two adjacent gaze points with the same angle and angle mapped on the visualization graph are extended on the lines with the same angle and angle mapped on the visualization graph by the unit length.

In step 140, a visualization graph of the frequency of eye movement scanning direction is formed by connecting the vertices of the extended lines corresponding to individual angles in the visualization graph.

In step 140, a radar chart is used as the visualization graph, and the visualization graph of the eye movement scanning direction frequency is synchronously mapped with the corresponding collected eye movement data to form a dynamic visualization graph of the eye movement scanning direction frequency. Based on the collected eye movement data, the gaze trajectory map obtained by connecting the collected gaze points in order is shown in FIG. 8 (a), as well as the real-time visualization of the frequency of eye movement scanning direction, as shown in FIG. 8 (b). The frequency of each eye movement and scanning direction during the observation of visual field information by visualizing graphics is visually viewed.

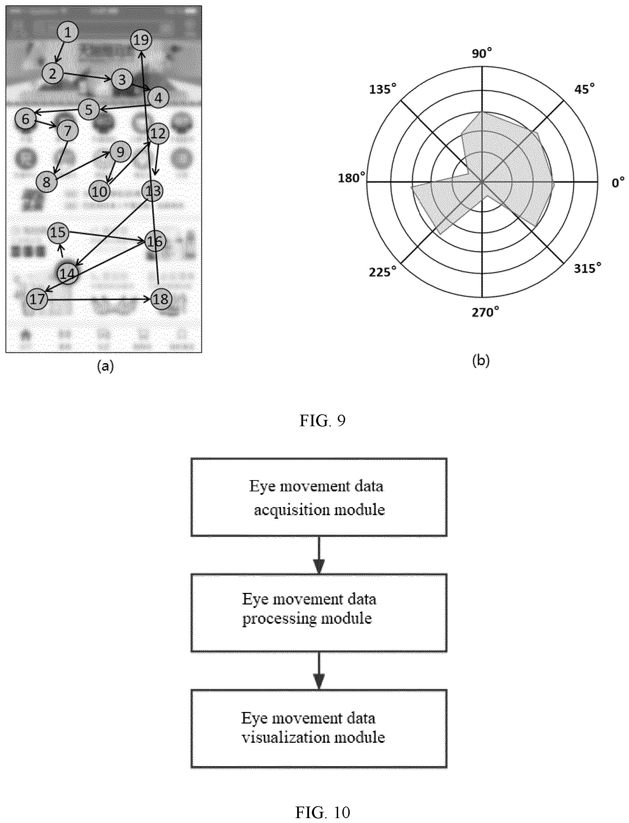

In one embodiment, the method described in the present application is applied to the design of an APP interface. With the eye movement data collected by the eye tracker, two adjacent gaze points are connected in sequence according to the movement order of the gaze points of the eyes in the APP interface during the subject's browsing process. The real-time trajectory of the gaze points of the eyes in the APP interface is obtained, as shown in FIG. 9 (a), and the visualization graph of the frequency of eye movement scanning direction when the eyes browse the APP interface is shown in FIG. 9 (b).

The visualization graph of eye movement scanning frequency formed in the above embodiments can reflect the visual characteristics of the subject's eye movement scanning while browsing the APP interface, in order to judge the rationality and reading efficiency of the APP interface design, and thus optimize the APP interface design accordingly. For example, in a certain APP interface, the user needs to find the specific content elements at the bottom of the page through the prompts of the top elements on the page. At this time, the expected eye movement scanning direction feature is mainly top to bottom. Therefore, the presentation range in the visualization graphs should be mainly top to bottom. If the presentation range of the left and right areas is too large, it means that the design of the APP interface has a significant impact on the efficiency of the user's browsing and searching during the downward browsing process, and the interface design is not reasonable enough and needs to be optimized.

The method described in this invention can be applied not only to APP interface design, but also to applications in fields such as architectural design and environmental behavior, industrial engineering and safety engineering, industrial design, traffic safety, military defense, etc., by visualizing the frequency of eye movement scanning direction. In the field of traffic safety, it can be applied to the design of traffic signs, lanes, and other signs. Visualization graphs can be used to observe whether the direction of the driver's eye movement changes significantly when watching a certain traffic sign, and whether there is a significant change in a certain direction. If there is a significant change in a certain direction, it may mean that in general, some information in that direction on the sign affects the driver's attention to the central important information, which has a distracting effect on the driver's attention and affects driving safety. It needs to be adjusted and optimized to a relatively balanced state of eye movement attention.

Correspondingly to the above method, the present application further provides an eye movement data visualization device based on gaze trajectory, including a processor and a memory, wherein the memory stores computer instructions, and the processor is used to execute the computer instructions stored in the memory. When the computer instructions are executed by the processor, the system implements the steps of the eye movement data visualization method as described above.

The above eye movement data visualization device based on gaze trajectory, as shown in FIG. 10, includes:

eye movement data acquisition module, which collects eye movement data through any type of eye tracker.

eye movement data processing module, when receiving instructions for visualizing eye movement data, processing the obtained eye movement data to obtain eye movement features such as gaze point position, gaze point sequence, and gaze point trajectory when the subject observes the application scenario.

eye movement data visualization module, determining the positional relationship between adjacent gaze points according to the gaze point sequence in the gaze point trajectory, connecting every two adjacent gaze points, and determining the angle between the line connecting the two adjacent gaze points and the predetermined reference axis; based on the included angle between the line connecting two adjacent gaze points and a predetermined reference axis, mapping the line connecting the two adjacent gaze points to a visualization graph at the included angle, wherein, when mapping, if there are already lines with the same included angle mapped on the visualization graph, the line connecting the two adjacent gaze points extends along the line with the same angle mapped on the visualization graph by a unit length; and, by connecting the vertices of the extended lines corresponding to individual angles in the visualization graph, forming a visualization graph of the frequency of eye movement scanning direction.

A gaze trajectory based eye movement data visualization device in one embodiment, corresponding to the gaze trajectory based eye movement data visualization method, uses the X-axis of a plane Cartesian coordinate system established with the previous gaze point in every two adjacent gaze points as the origin as the predetermined reference axis; and use radar charts as visualization graphs.

The embodiments of the present application also provide a computer-readable storage medium on which a computer program is stored, characterized in that the program, when executed by a processor, implements the steps of the eye movement data visualization method as described above. The computer-readable storage medium may be a tangible storage medium, such as random access memory (RAM), memory, read-only memory (ROM), electrically programmable ROM, electrically erasable programmable ROM, registers, floppy disks, hard disks, removable storage disks, CD ROMs, or any other form of storage medium known in the art.

Ordinary technical personnel in this field should be able to understand that the various exemplary components, systems, and methods described in conjunction with the embodiments disclosed herein can be implemented in hardware, software, or a combination of both. Whether to execute it in hardware or software depends on the specific application and design constraints of the technical solution. Professional technicians can use different methods to achieve the described functions for each specific application, but such implementation should not be considered beyond the scope of the present application. When implemented in hardware, it can be, for example, electronic circuits, application specific integrated circuits (ASICs), appropriate firmware, plugins, function cards, and so on. When implemented in software, the elements of the present application are programs or code segments used to perform the required tasks. Programs or code segments can be stored in machine-readable media, or transmitted over transmission media or communication links through data signals carried on carriers.

It should be clarified that the present application is not limited to the specific configuration and processing described above and shown in the Drawings. For the sake of simplicity, detailed descriptions of known methods are omitted here. In the above embodiments, several specific steps are described and illustrated as examples. However, the method process of the present application is not limited to the specific steps described and illustrated. Those skilled in the art may make various changes, modifications, additions, or alter the order of steps after understanding the spirit of the present application.

In the present application, features described and/or illustrated for one embodiment may be used in the same or similar manner in one or more other embodiments, and/or combined with or substituted for features of other embodiments.

The above description is only a preferred embodiment of the present application and is not intended to limit the present application. For those skilled in the art, various modifications and variations can be made to the embodiments of the present application. Any modifications, equivalent substitutions, improvements, etc. made within the spirit and principles of the present application shall be included within the scope of protection of the present application.

**Claims**

1. A method for visualizing eye movement data based on gaze trajectory, **characterized by** comprising the following steps:

collecting eye movement data of a subject, and based on the collected eye movement data, obtaining gaze point position, gaze point sequence, and gaze point trajectory of the subject;
determining a positional relationship between adjacent gaze points according to the gaze point sequence in the gaze point trajectory, connecting every two adjacent gaze points, and determining degree of an included angle between the line connecting the two adjacent gaze points and a predetermined reference axis;
based on the degree of an included angle between the line connecting two adjacent gaze points and the predetermined reference axis, mapping the line connecting the two adjacent gaze points to a visualization graph via the included angle, wherein, when mapping, if there are already a line with the same included angle mapped on the visualization graph, the line connecting the two adjacent gaze points extends along the line with the same angle mapped on the visualization graph by a unit length; and
forming the visualization graph of a frequency of eye movement scanning direction by connecting vertices of extended lines corresponding to individual angles in the visualization graph.

2. The method according to claim 1, **characterized in that**, a step of determining the degree of the included angle between the line connecting two adjacent gaze points and the predetermined reference axis comprises: establishing a plane Cartesian coordinate system with a previous gaze point of two adjacent gaze points as an origin, determining a position coordinate of a latter gaze point of the two adjacent gaze points in the plane Cartesian coordinate system, and then determining degree of an included angle between the line connecting two adjacent gaze points and a predetermined coordinate axis in the plane Cartesian coordinate system.

3. The method according to claim 2, **characterized in that**, a step of determining the degree of the included angle between the line connecting two adjacent gaze points and the predetermined coordinate axis in the plane Cartesian coordinate system comprises: based on a plane Cartesian coordinate system with a previous gaze point of two adjacent gaze points as an origin, determining a quadrant coordinate of a latter gaze point of the two adjacent gaze points in the plane Cartesian coordinate system, and determining a degree of an included angle between the line connecting two adjacent gaze points and an X-axis of the plane Cartesian coordinate system counterclockwise.

4. The method according to claim 3, **characterized in that**, a step of determining the degree of the included angle between the line connecting two adjacent gaze points and the X-axis of the plane Cartesian coordinate system counterclockwise comprises: converting a quadrant coordinate of a latter gaze point of every two adjacent gaze points into a coordinate in a first quadrant through axis symmetry or center symmetry, and measuring the degree of the included angle between the line connecting two adjacent gaze points after conversion and the X-axis of the plane Cartesian coordinate system counterclockwise; and based on the quadrant coordinate of the latter gaze point in the plane Cartesian coordinate system, determining the degree of the included angle between the line connecting two adjacent gaze points and the X-axis of the plane Cartesian coordinate system counterclockwise.

5. The method according to claim 4, **characterized in that**, a formula for calculating the degree of the included angle between the line connecting two adjacent gaze points after conversion and the X-axis of the plane Cartesian coordinate system counterclockwise is:

$$\text{Arctan}\theta' = (|y_2|-y_1)/(|x_2|-x_1)$$

where a previous gaze point is used as a first eye movement gaze point and a latter gaze point is used as a second eye movement gaze point; $\theta'$ is degree of the included angle between the line connecting two adjacent gaze points after conversion and the X-axis of the plane Cartesian coordinate system counterclockwise; $X_1$ is a horizontal ordinate of a first eye movement gaze point; $X_2$ is the horizontal ordinate of a second eye movement gaze point; Yi is a vertical ordinate of the first eye movement gaze point; $Y_2$ is a vertical ordinate of the second eye movement gaze point; $|Y_2|$ is a vertical coordinate of the second eye movement gaze point after being converted into the first quadrant; and $|X_2|$ is a horizontal coordinate of the second eye movement gaze point after being converted into the first quadrant.

6. The method according to claim 4, **characterized in that**, a step of determining the degree of the included angle between the line connecting two adjacent gaze points and the X-axis of the plane Cartesian coordinate system counterclockwise based on the quadrant coordinates of the latter gaze point in the plane Cartesian coordinate system comprises: when the coordinate of the latter gaze point is located in the first quadrant, obtaining the degree of the included angle of $\theta'$; when the coordinate of the latter gaze point is within the second quadrant, obtaining the degree of the included angle of $180° - \theta'$; when the coordinate of the latter gaze point is located in the third quadrant, obtaining the degree of the included angle of $180°+\theta'$; and when the coordinate of the latter gaze point is located in the fourth quadrant, obtaining the degree of the included angle of $360° - \theta'$.

7. The method according to claim 1, **characterized in that**, the visualization graph of the frequency of eye movement scanning direction corresponds to the collected eye movement data and is synchronously mapped to form a dynamic visualization graph of the frequency of eye movement scanning direction.

8. The method according to claim 1, **characterized in that**, the collected eye movement data is collected through an eye tracker.

9. An eye movement data visualization device based on gaze trajectory, **characterized by** comprising a processor and a memory, wherein the memory stores computer instructions, the processor is used to execute the computer instructions stored in the memory, and when the computer instructions are executed by the processor, the system implements the steps of any one of the methods according to claims 1 to 8.

10. A computer-readable storage medium storing a computer program, **characterized in that**, the program, when executed by a processor, implements the steps of any one of the methods according to claims 1 to 8.

collecting eye movement data of a subject, and based on the collected eye movement data, obtaining gaze point position, gaze point sequence, and gaze point trajectory of the subject;

S110

determining a positional relationship between adjacent gaze points according to the gaze point sequence in the gaze point trajectory, connecting every two adjacent gaze points, and determining degree of an included angle between the line connecting the two adjacent gaze points and a predetermined reference axis;

S120

based on the degree of an included angle between the line connecting two adjacent gaze points and the predetermined reference axis, mapping the line connecting the two adjacent gaze points to a visualization graph via the included angle, wherein, when mapping, if there are already a line with the same included angle mapped on the visualization graph, the line connecting the two adjacent gaze points extends along the line with the same angle mapped on the visualization graph by a unit length; and

S130

forming the visualization graph of a frequency of eye movement scanning direction by connecting vertices of extended lines corresponding to individual angles in the visualization graph

S140

FIG. 1

Obtaining sequence of previous and latter gaze
points in eye movement

Establishing a plane Cartesian coordinate system
with a first gaze point as an origin, and determining
position coordinate of a second gaze point

Judging the quadrant where
the coordinate of the second
gaze point is, and selecting
different calculating method

First
quadrant

Second
quadrant

Third
quadrant

Fourth
quadrant

Outputting degree of θ
angle

Mapping to radar chart by one
unit length

FIG. 2

$(x_2, y_2)$

$\theta = 45°$

$(x_1, y_1)$

FIG. 3

$$\theta = 180° - \theta'$$

FIG. 4

$$\theta = \theta' + 180°$$

FIG. 5

$$\theta = 360° - \theta'$$

FIG. 6

FIG. 7

(a)

(b)

FIG. 8

(a)

(b)

FIG. 9

Eye movement data
acquisition module

Eye movement data
processing module

Eye movement data
visualization module

FIG. 10

## INTERNATIONAL SEARCH REPORT

| | International application No. |
|---|---|
| | **PCT/CN2023/139927** |

**A. CLASSIFICATION OF SUBJECT MATTER**

G06F3/01(2006.01)i

According to International Patent Classification (IPC) or to both national classification and IPC

**B. FIELDS SEARCHED**

Minimum documentation searched (classification system followed by classification symbols)

IPC: G06F3/-

Documentation searched other than minimum documentation to the extent that such documents are included in the fields searched

Electronic data base consulted during the international search (name of data base and, where practicable, search terms used)

CNTXT, CNKI, WPABS, DWPI, ENTXT: 北京津发科技, 赵起超, 杨苒, 眼动, 可视化, 数据, 雷达, 角度, 视点, 轨迹, eye+, mov+, visual+, fixa+, track+, connect+, line+, angle+

**C. DOCUMENTS CONSIDERED TO BE RELEVANT**

| Category* | Citation of document, with indication, where appropriate, of the relevant passages | Relevant to claim No. |
|---|---|---|
| PX | CN 116594496 A (KINGFAR INTERNATIONAL INC.) 15 August 2023 (2023-08-15) description, paragraphs [0004]-[0059], and figures 1-10 | 1-10 |
| Y | CN 113425247 A (BEIJING UNIVERSITY OF POSTS AND TELECOMMUNICATIONS) 24 September 2021 (2021-09-24) description, paragraphs [0005]-[0118], and figures 1-7 | 1-10 |
| Y | CN 108921109 A (ANHUI FUSION INTELLIGENT TECHNOLOGY CO., LTD.) 30 November 2018 (2018-11-30) description, paragraphs [0007]-[0081], and figure 1 | 1-10 |
| A | CN 107679048 A (BOYUAN SENHE INFORMATION SCIENCE & TECHNOLOGY (BEIJING) CO., LTD.) 09 February 2018 (2018-02-09) entire document | 1-10 |
| A | CN 108932473 A (CHINA CONSTRUCTION BANK CORP.) 04 December 2018 (2018-12-04) entire document | 1-10 |
| A | CN 111949131 A (CHEN TAO) 17 November 2020 (2020-11-17) entire document | 1-10 |

☑ Further documents are listed in the continuation of Box C. ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **21 March 2024** | **26 March 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

| International application No. |
| --- |
| **PCT/CN2023/139927** |

**C.      DOCUMENTS CONSIDERED TO BE RELEVANT**

| Category* | Citation of document, with indication, where appropriate, of the relevant passages | Relevant to claim No. |
| --- | --- | --- |
| A | CN 114795104 A (ZHENGZHOU UNIVERSITY) 29 July 2022 (2022-07-29)<br>    entire document | 1-10 |
| A | JP 2005065716 A (NAGAYA, M.) 17 March 2005 (2005-03-17)<br>    entire document | 1-10 |

Form PCT/ISA/210 (second sheet) (July 2022)

**INTERNATIONAL SEARCH REPORT**
Information on patent family members

International application No.

**PCT/CN2023/139927**

| Patent document cited in search report | | | Publication date (day/month/year) | Patent family member(s) | | | Publication date (day/month/year) |
|---|---|---|---|---|---|---|---|
| CN | 116594496 | A | 15 August 2023 | None | | | |
| CN | 113425247 | A | 24 September 2021 | CN | 113425247 | B | 23 December 2022 |
| CN | 108921109 | A | 30 November 2018 | CN | 108921109 | B | 17 September 2021 |
| CN | 107679048 | A | 09 February 2018 | None | | | |
| CN | 108932473 | A | 04 December 2018 | None | | | |
| CN | 111949131 | A | 17 November 2020 | None | | | |
| CN | 114795104 | A | 29 July 2022 | None | | | |
| JP | 2005065716 | A | 17 March 2005 | JP | 4406230 | B2 | 27 January 2010 |

Form PCT/ISA/210 (patent family annex) (July 2022)

**REFERENCES CITED IN THE DESCRIPTION**

**Patent documents cited in the description**

- CN 202211705807